(19) Europäisches Patentamt
European Patent Office
Office européen des brevets

(11) Veröffentlichungsnummer : **0 041 170**
**B1**

(12) # EUROPÄISCHE PATENTSCHRIFT

(45) Veröffentlichungstag der Patentschrift : **25.07.84**

(51) Int. Cl.³ : **C 07 C103/375**, C 07 C103/68, C 07 C119/12, G 03 C 7/32

(21) Anmeldenummer : **81103787.8**

(22) Anmeldetag : **18.05.81**

(54) **3-Nitro-pivaloyl-acetanilide, Verfahren zu ihrer Herstellung und ihre Verwendung.**

(30) Priorität : **29.05.80 DE 3020443**

(43) Veröffentlichungstag der Anmeldung : **09.12.81 Patentblatt 81/49**

(45) Bekanntmachung des Hinweises auf die Patenterteilung : **25.07.84 Patentblatt 84/30**

(84) Benannte Vertragsstaaten : **DE FR GB**

(56) Entgegenhaltungen :
**CH-A- 267 110**
**DE-B- 1 004 163**
**DE-C- 256 621**

(73) Patentinhaber : **BAYER AG**
**Konzernverwaltung RP Patentabteilung**
**D-5090 Leverkusen 1 Bayerwerk (DE)**

(72) Erfinder : **Blank, Heinz Ulrich, Dr.**
**Am Geusfelde 35**
**D-5068 Odenthal (DE)**
Erfinder : **Wolters, Erich, Dr.**
**Streffenweg 22**
**D-5162 Niederzier (DE)**
Erfinder : **Müller, Karl Walter, Dr.**
**Bertha-von-Suttner-Strasse 29**
**D-5090 Leverkusen (DE)**
Erfinder : **Rottloff, Günter**
**Semmelweisstrasse 70**
**D-5000 Koeln 80 (DE)**

**EP 0 041 170 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 3-Nitro-pivaloyl-acetanilide, zwei Verfahren zu ihrer Herstellung und ihre Verwendung zur Synthese von Kupplungskomponenten für die Farbfilmfotographie sowie Zwischenprodukte bei einem der Herstellungsverfahren.

Aus der DE-AS 10 04 163 ist bekannt, Diketen mit substituierten aromatischen Aminen mit kleiner Dissoziationskonstante unter Zusatz kleiner Mengen eines basischen tertiären Amins umzusetzen.

Weiterhin ist es aus DE-PS 256 621 bekannt, o-Halogen-, o-Alkoxy-, o-Aralkyloxy- oder o-Aryloxyaniline durch Erhitzen mit Acetessigester in die entsprechenden Acetessigsäureanilide umzusetzen.

Es wurden die 3-Nitro-pivaloyl-acetanilide der Formel (I)

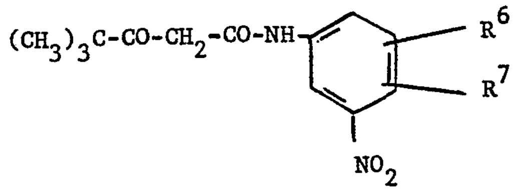

(I)

gefunden, in der

$R^6$ und $R^7$ unabhängig voneinander für Wasserstoff, Methyl, Ethyl, Methoxy, Methylthio, Chlor, Brom, Nitro, Dimethylamino, Diethylamino oder Benzyl-methylamino stehen.

Unter den erfindungsgemäßen Verbindungen sei besonders das 2-Chlor-5-nitro-pivaloyl-acetanilid der Formel (II)

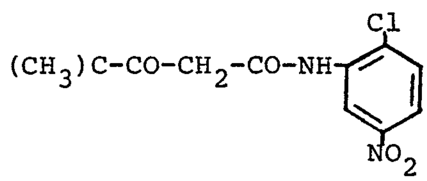

(II)

genannt.

Es wurde weiterhin ein Verfahren zur Herstellung der 3-Nitro-pivaloyl-acetanilide der Formel (I)

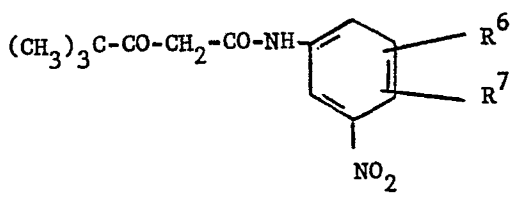

(I)

in der

$R^6$ und $R^7$ die genannte Bedeutung haben,

gefunden, das dadurch gekennzeichnet ist, daß man einen Pivaloylessigsäureester der Formel (III)

$$(CH_3)_3C—CO—CH_2—COR^9 \qquad (III)$$

in der

$R^9$ Methoxy oder Ethoxy bedeutet,

mit 3-Nitroanilinen der Formel (IV)

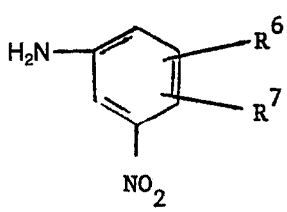

(IV)

in der

$R^6$ und $R^7$ die oben angegebene Bedeutung haben,

bei einer Temperatur von 50 bis 200 °C, gegebenenfalls in Gegenwart eines inerten Verdünnungsmittels, kondensiert.

Als Pivaloylessigsäureester kann der Methyl- oder der Ethylester eingesetzt werden. In bevorzugter Weise wird der Methylester eingesetzt.

Pivaloylessigsäureester für das erfindungsgemäße Verfahren sind dem Fachmann bekannt. So kann beispielsweise der Pivaloylessigsäuremethylester durch Erhitzen unter Decarbonylierung aus Pivaloylbrenztraubensäuremethylester hergestellt werden (US 2 527 306).

Als 3-Nitroaniline für das erfindungsgemäße Verfahren können solche der Formel (IV) eingesetzt werden.

Bevorzugt wird das 2-Chlor-5-nitro-anilin eingesetzt.

Das erfindungsgemäße Verfahren kann am Beispiel der Kondensation von Pivaloylessigsäuremethylester und 2-Chlor-5-nitroanilin durch die folgende Formelgleichung erläutert werden :

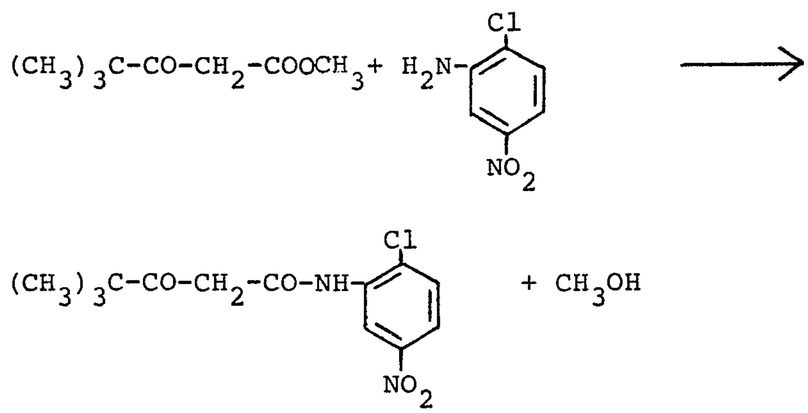

Zur Umsetzung erfordern die beiden Reaktionspartner ein molares Verhältnis von 1 : 1. Die Reaktion des erfindungsgemäßen Verfahrens läuft jedoch auch bei einem anderen molaren Verhältnis bis zum Verbrauch der jeweils im Unterschuß vorhandenen Verbindung ab. Zur Erleichterung der Aufarbeitung des Reaktionsgemisches ist es jedoch vorteilhaft, den Pivaloylessigsäureester in einer mindestens molaren Menge, bezogen auf das 3-Nitroanilin, einzusetzen, um eine maximale Umsetzung des Nitroanilins, das sich nur schwer aus dem Endprodukt entfernen läßt, anzustreben. Als mindestens molare Menge des Pivaloylessigsäureesters sei beispielsweise ein molares Verhältnis von Pivaloylessigsäureester zu 3-Nitroanilin von 1 : 1 bis 1,5 : 1, bevorzugt 1,1 : 1 bis 1,2 : 1, genannt.

Das erfindungsgemäße Verfahren wird bei einer Temperatur von 50 bis 200, bevorzugt 100 bis 180, besonders bevorzugt 120 bis 150° durchgeführt. Das erfindungsgemäße Verfahren ist vom Außendruck unabhängig und wird gewöhnlich bei Normaldruck durchgeführt. Es ist dem Fachmann jedoch bekannt, beim Einsatz eines unterhalb der Reaktionstemperatur siedenden Lösungsmittels bei geringem Überdruck zu arbeiten oder zur Einstellung eines Rückflusses bei einem oberhalb der Reaktionstemperatur siedenden Lösungsmittels unter geringem Unterdruck zu arbeiten.

Das erfindungsgemäße Verfahren kann mit oder ohne Verdünnungsmittel durchgeführt werden. Für den Fall, daß ein Verdünnungsmittel benutzt wird, muß dieses inert gegenüber den Reaktionspartnern sein. Als solche inerten Verdünnungsmittel seien beispielsweise aliphatische oder aromatische Kohlenwasserstoffe oder deren Halogensubstitutionsprodukte genannt, wie 2,2,4-Trimethylpentan, Benzinfraktionen mit geeignetem Siedebereich, Toluol, Xylol, Chlorbenzol oder Dichlorbenzol. Bevorzugt wird ein solches Verdünnungsmittel eingesetzt, das mit dem abgespaltenen Alkohol azeotrop abdestilliert, wie Heptan, Octan, Dichloräthan, Trichloräthan, Tetrachloräthan, Toluol oder Äthylbutyläther, bevorzugt Toluol. Hierbei kann sodann der Fortschritt der Kondensationsreaktion an der Temperatur am Kopf einer auf das Reaktionsgefäß gesetzten Destillationskolonne verfolgt werden. Diese Temperatur am Kopf der Kolonne zeigt in diesem Fall bis zur Beendigung der Kondensation die Siedetemperatur des Azeotrops aus dem abgespaltenen Alkohol und dem bevorzugt eingesetzten Lösungsmittel an und steigt danach auf die Siedetemperatur des eingesetzten Lösungsmittels.

Bei der bevorzugten Verfahrensvariante der Kondensation von Pivaloylessigsäuremethyl- oder -ethylester mit 2-Chlor-5-nitroanilin kann vorteilhaft ein Gemisch von 1 Mol des Anilins, 1,2 Mol des Pivaloylessigsäureesters und etwa 2 Mol Toluol als Verdünnungsmittel unter Rühren zum Sieden des Reaktionsgemisches erhitzt werden. Der gebildete Alkohol wird sodann als Azeotrop mit Toluol abdestilliert, wobei die Kopftemperatur der Destillationskolonne längere Zeit den Siedepunkt des Azeotrops zeigt, der im Beispiel des Methylesters und Azeotrops Methanol/Toluol etwa 64 °C beträgt. Die Reaktion wird dann fortgesetzt, bis die Kopftemperatur der Kolonne den Siedepunkt des Toluols mit etwa 110 °C zeigt. Die auf diese Weise erreichbare Ausbeute liegt nach der Isolierung des Produkts bei mindestens 90 % der theoretischen Ausbeute, bezogen auf das eingesetzte Nitroanilin, in vielen Fällen über 95 % der theoretischen Ausbeute. Die Aufarbeitung erfolgt durch destillative Entfernung des Verdünnungsmittels und des Überschusses an Pivaloylessigsäureester. Daraufhin wird entweder der verbleibende Destillationssumpf erneut mit einem der genannten Verdünnungsmittel versetzt und das

Produkt nach Abkühlung als Kristallisat gewonnen oder die nach Destillation des Verdünnungsmittels und des überschüssigen Pivaloylessigsäureesters erhaltene Produktschmelze wird direkt auf eine Kühlwalze gegeben und als schuppenförmiges Produkt gewonnen. Bei dieser letzteren Aufarbeitungsvariante sind Ausbeuten bis zu 99 % der theoretischen Ausbeute möglich.

Die vorliegende Erfindung betrifft ferner ein weiteres Verfahren zur Herstellung von 3-Nitro-pivaloylacetaniliden der Formel (I)

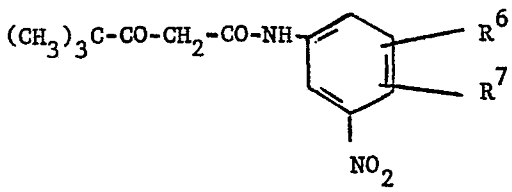

(I)

in der

$R^6$ und $R^7$ die oben genannte Bedeutung haben, das dadurch gekennzeichnet ist, daß man ein Enamin der Formel (V)

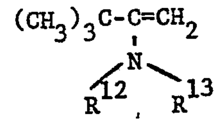

(V)

in der

$R^{12}$ und $R^{13}$ unabhängig voneinander $C_1$-$C_4$-Alkyl, Benzyl oder Phenyl bedeuten oder

$R^{12}$ und $R^{13}$ gemeinsam mit dem Stickstoffatom, das sie substituieren, einen 5- bis 7-gliedrigen stickstoffhaltigen Heterocyclus bedeuten, der gegebenenfalls weitere Heteroatome enthalten kann, mit einem 3-Nitro-phenylisocyanat der Formel (VI)

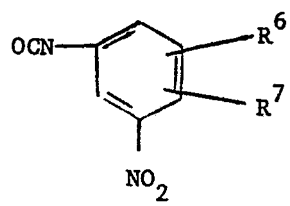

(VI)

in der

$R^6$ und $R^7$ die angegebene Bedeutung haben,

bei einer Temperatur von − 5 °C bis + 100 °C, gegebenenfalls in Gegenwart eines inerten Verdünnungsmittels so umsetzt, daß das Enamin stets im Überschuß vorliegt, und das als Zwischenprodukt entstehende β-Amino-γ,γ-dimethyl-penten-(2)-carbonsäure-3-nitro-anilid der Formel (VII)

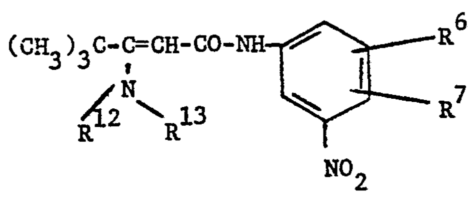

(VII)

in der

$R^6$, $R^7$, $R^{12}$ und $R^{13}$ die angegebene Bedeutung haben,

gegebenenfalls nach vorheriger Isolierung und Reinigung, hydrolisiert.

Als $C_1$-$C_4$-Alkyl sei beispielsweise Methyl, Ethyl, Propyl oder Butyl, bevorzugt Methyl genannt. Für den Fall, daß $R^{12}$ und $R^{13}$ gemeinsam mit dem Stickstoffatom, das sie substituieren, einen stickstoffhaltigen Heterocyclus bedeuten, sei beispielsweise ein 5- bis 7-gliedriger Heterocyclus genannt, wie Pyrrolidin, Piperidin oder Azepin. Dieser Stickstoffhaltige Heterocyclus kann weitere Heteroatome, wie Stickstoff, Schwefel oder Sauerstoff enthalten. Beispielsweise seien hierzu die folgenden Systeme genannt: Pyrazolidin, Pyrazolin, Imidazolin, Imidazolidin, Oxazolin, Oxazolidin, Thiazolin, Thiazolidin, Piperazin, Morpholin oder Thiomorpholin.

Für das Verfahren geeignete Enamine können auf bekannte Weise aus den zugehörigen Carbonylverbindungen und sekundären Aminen, beispielsweise Dimethylamin, Pyrrolidin, Piperidin oder Morpholin, und Titantetrachlorid hergestellt werden (J. Org. Chem. *32,* 213 (1967)).

Bevorzugt werden im erfindungsgemäßen Verfahren Enamine der Formel (VIII)

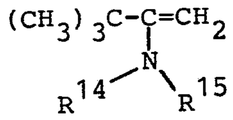

(VIII)

eingesetzt, in der

$R^{14}$ und $R^{15}$ Methyl bedeuten oder gemeinsam mit dem Stickstoffatom, das sie substituieren, das System Pyrrolidin, Piperidin oder Morpholin bedeuten.

In besonders bevorzugter Weise wird das 3,3-Dimethyl-2-dimethylamino-1-buten der Formel (IX)

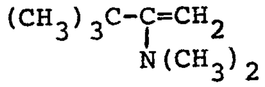

(IX)

eingesetzt.

Die 3-Nitro-phenylisocyanate der Formel (VI) können beispielsweise nach einem bisher nicht veröffentlichten Verfahren durch Phosgenierung der entsprechend substituierten 3-Nitro-aniline hergestellt werden.

Im erfindungsgemäßen Verfahren wird bevorzugt das 2-Chlor-5-nitro-phenylisocyanat eingesetzt.

Das erfindungsgemäße Verfahren kann am Beispiel der Umsetzung von 3,3-Dimethyl-2-dimethylamino-1-buten mit 2-Chlor-5-nitro-phenylisocyanat und Hydrolyse des entstehenden β-Dimethylamino-γ,γ, dimethyl-penten(2)-carbonsäure-(2-chlor-5-nitro)-anilid durch die folgenden Formelgleichungen erläutert werden :

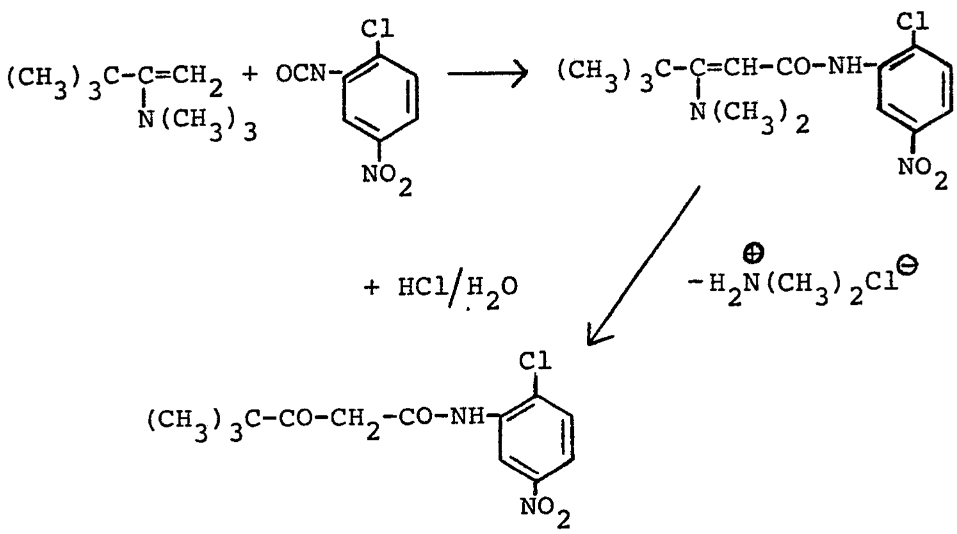


Das erfindungsgemäße Verfahren wird bei einer Temperatur von − 5 °C bis + 100 °C, bevorzugt 0 bis 80 °C, besonders bevorzugt bei etwa Raumtemperatur, durchgeführt.

Das erfindungsgemäße Verfahren kann mit oder ohne Verdünnungsmittel durchgeführt werden. Für den Fall, daß ein Verdünnungsmittel eingesetzt wird, muß dieses gegenüber den Reaktionspartnern inert sein. Geeignete inerte Lösungsmittel sind solche, die kein bewegliches Wasserstoffatom tragen, wie Kohlenwasserstoffe, chlorierte Kohlenwasserstoffe oder Ether. Als Beispiele seien genannt : Benzinfraktionen, Cyclohexan, Benzol, Toluol, Xylol, Methylenchlorid, Chlorbenzol, Dichlorbenzol, Diethylether, Tetrahydrofuran oder Dioxan. Als Verdünnungsmittel kann weiterhin auch überschüssiges Enamin dienen, das bei der Aufarbeitung des Reaktionsgemisches zuruckgewonnen werden kann.

Die Reaktion des erfindungsgemäßen Verfahrens erfordert zur Vervollständigung ein molares Verhältnis des Enamins und des Phenylisocyanats von 1 : 1. Zur Vermeidung unerwünschter Nebenreaktionen wird das erfindungsgemäße Verfahren jedoch so durchgeführt, daß das Enamin im Reaktionsgemisch stets im Überschuß, vorhanden ist. Lediglich beim Abschluß der Reaktion des erfindungsgemäßen Verfahrens kann wieder ein äquimolares Verhältnis der umgesetzten Reaktionspartner erreicht werden. Der molare Überschuß des Enamins über das Phenylisocyanat kann beliebig sein, insbesondere bei der Verwendung überschüssigen Enamins als Lösungsmittel. Beispielsweise sei ein Bereich von 1 bis 5 Molen Enamin pro Mol Phenylisocyanat genannt.

Zur Aufrechterhaltung eines Enamin-Uberschusses im Reaktionsgemisch wird die Reaktion so durchgeführt, daß man zu dem vorgelegten reinen oder verdünnten Enamin das Isocyanat in reiner oder verdünnter Form in stöchiometrischer oder in weniger als stöchiometrischer Menge zufügt. Die Zugabe des Isocyanats kann rasch erfolgen, da die Reaktion des erfindungsgemäßen Verfahrens schnell abläuft. Selbstverständlich kann auch nur ein Teil des Enamins vorgelegt werden und das restliche Enamin mit

dem Phenylisocyanat so zugegeben werden, daß zu jedem Zeitpunkt der Reaktion ein Uberschuß des Enamins im Reaktionsgemisch vorliegt.

Für den Fall, daß man in Gegenwart eines Verdünnungsmittels arbeitet, kann sowohl das vorgelegte Enamin, als auch das zugegebene Phenylisocyanat unabhängig voneinander in reiner Form oder in verdünnter Form eingesetzt werden. Eine bevorzugte Variante des erfindungsgemäßen Verfahrens benutzt eine etwa 20 bis 25 gew.-%ige Lösung des Isocyanats, wie sie bei der Herstellung dieses Isocyanats durch Heißphosgenierung anfällt. Beispielsweise kann dies eine Lösung des Isocyanats in Chlorbenzol sein. Bei dieser Verfahrensweise wird das Enamin ohne weiteres Verdünnungsmittel vorgelegt.

Zur nachfolgenden Hydrolyse des zunächst entstehenden Zwischenproduktes wird dieses mit mindestens der äquivalenten Menge Wasser umgesetzt. Für einen zügigen Verlauf der Hydrolyse und zur Vermeidung von störenden Nebenreaktionen ist es vorteilhaft, hierbei einen pH-Bereich von 4 bis 9 einzuhalten. Dies geschieht durch dosierte Zugabe einer Säure oder einer Base zum Reaktionsgemisch. Zur Vermeidung unerwünschter Ionen im Reaktionsgemisch ist es vorteilhaft, einfache Mineralsäuren, wie Salzsäure, Schwefelsäure oder Phosphorsäure einzusetzen. Bevorzugt wird im schwach sauren Bereich bei pH 4 bis pH 6, besonders bevorzugt in der Nähe des pH-Wertes 5, gearbeitet. Als hierbei einzusetzende Säure wird bevorzugt Salzsäure benutzt.

Die Hydrolyse des Zwischenproduktes bei Benutzung von Salzsäure kann durch Zugabe der benötigten Menge Wasser und anschließendes Zutropfen von konzentrierter wäßriger Salzsäure durchgeführt werden, wobei die Zutropfgeschwindigkeit so geregelt wird, daß ein pH-Wert von etwa 5 aufrechterhalten wird. Zur Isolierung des gewünschten Produktes kann nach Phasentrennung und gegebenenfalls nach Filtration nach üblichen Methoden gearbeitet werden, beispielsweise durch Abkühlen und/oder Einengen der organischen Phase und Kristallisation des Reaktionsproduktes. Eine andere vorteilhafte Variante der Hydrolyse ist dadurch gekennzeichnet, daß man gasförmigen Chlorwasserstoff in das Reaktionsgemisch einleitet und damit das zunachst im erfindungsgemäßen Verfahren entstehende Zwischenprodukt als Immoniumsalz ausfällt. Nach der völligen oder teilweisen Entfernung des organischen Lösungsmittels kann das gefällte Immoniumsalz durch Verrühren mit Wasser und gegebenenfalls unter Zusatz basischer Stoffe, wie wäßrige Natronlauge, bei einem pH-Wert von etwa 5 zum gewünschten Entprodukt hydrolysiert werden. Diese Variante der Hydrolyse kann vorteilhaft durchgeführt werden, wenn in Gegenwart eines der genannten Verdünnungsmittel gearbeitet wird.

Die Möglichkeit der Isolierung des entstehenden Zwischenproduktes in reiner Form oder in der Form des Immoniumsalzes gestattet es, eine zwischengeschaltete Reinigung, beispielsweise durch Umkristallisation, vorzunehmen und dadurch eine besonders hohe Reinheit des Endproduktes zu erreichen. Für den Fall, daß die Reinheit des Endproduktes ohne Zwischenisolierung und Reinigung des Zwischenproduktes für die weitere Verwendung ausreichend ist, kann jedoch auf diese Zwischenisolierung verzichtet werden.

Die Erfindung umfaßt somit auch die bei der Kondensation von Enaminen der Formel (V) mit 3-Nitrophenylisocyanaten der Formel (VI) entstehenden Zwischenprodukte in freier Form der Formel (VII) oder in Form ihrer Immoniumsalze der Salzsäure der Formel (X)

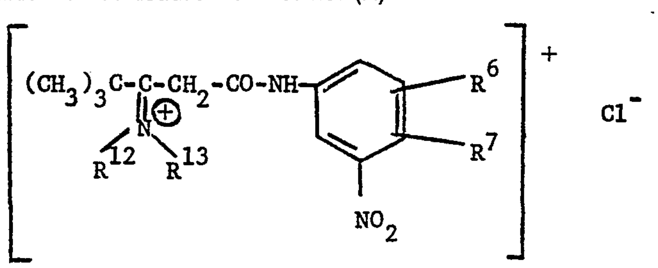

(X)

in der

$R^6$, $R^7$, $R^{12}$ und $R^{13}$ die angegebene Bedeutung haben.

Die im erfindungsgemäßen Verfahren aus einem Enamin und einem 3-Nitro-phenylisocyanat herstellbaren 3-Nitro-acetoacetanilide können in einer Ausbeute von etwa 90 bis 95 % der theoretischen Ausbeute an isoliertem Produkt gewonnen werden.

Die erfindungsgemäßen 3-Nitro-pivaloyl-acetanilide der Formel (I) können zur Synthese von Kupplungskomponenten für die Farbfotografie der Formel (XI)

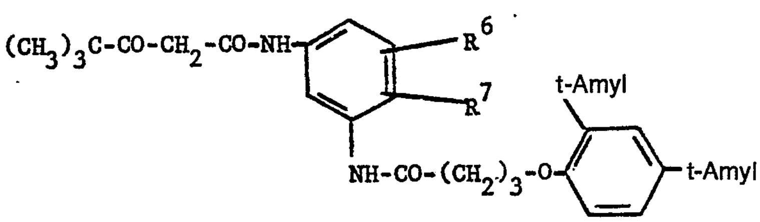

(XI)

in der

$R^6$ und $R^7$ die angegebene Bedeutung haben, verwendet werden.

Hierzu wird die Nitrogruppe des 3-Nitro-pivaloyl-acetanilids zur Aminogruppe reduziert und diese anschließend mit 2,4-Di-tert.-amyl-phenoxybuttersäurehalogenid unter Abspaltung von Halogenwasserstoff umgesetzt. So kann beispielsweise das 2-Chlor-5-nitro-pivaloyl-acetanilid durch Reduktion mit katalytisch angeregtem Wasserstoff zum 2-Chlor-5-amino-pivaloyl-acetanilid umgewandelt werden, das weiter mit 2,4-Di-tert.-amyl-phenoxybuttersäurechlorid zum Pivaloylessigsäure-2-chlor-5-(2,4-di-tert.-amyl-phenoxybutyramido)-anilid umgesetzt werden kann. Solche Verbindungen sind als Kupplungskomponente für die Farbfotografie bekannt (US 3 265 506, US 3 384 657, DE-AS 1 124 356).

## Beispiel 1

In einer 1-l-Rührapparatur mit einer 30 cm langen verspiegelten Vigreux-Kolonne mit Kolonnenkopf werden

137 g Pivaloylessigsäuremethylester und
130 g Toluol zusammen mit
124,5 g 2-Chlor-5-nitro-anilin

vorgelegt und unter Rühren zum Sieden erhitzt. Während 10 Stunden steigt die Sumpftemperatur von 120 bis 145 °C an. Die Übergangstemperatur steigt von 64 bis ca. 110 °C an.

Es werden während der Destillationszeit etwa 112 ml Toluol-Methanol-Gemisch, gleichmäßig ca. 10 bis 12 ml Destillat/h abgenommen.

Anschließend wird der Kolbenrückstand mit Eiswasser unter Rühren abgekühlt, der entstandene dicke Kristallbrei abgesaugt und 3 mal mit je ca. 30 ml kaltem Toluol gewaschen. Nach dem Trocknen im Vakuum bei 50 °C verbleiben 190 g (90,9 % der theoretischen Ausbeute, berechnet auf Nitranilin) an Pivaloyl-2-chlor-5-nitroanilid. Gelbe Kristalle ; Fp. 118 bis 119 °C.

Elementaranalyse :

| | | | | | |
|---|---|---|---|---|---|
| berechnet | C 52,3 % | H 5,0 % | N 9,4 % | Cl 11,9 % | O 21,5 % |
| gefunden | C 52,5 % | H 4,8 % | N 9,6 % | Cl 11,8 % | O 21,3 % |
| | C 52,4 % | H 4,7 % | N 9,6 % | Cl 11,7 % | O 21,6 % |

## Beispiel 2

Zu 5,5 g 3,3-Dimethyl-2-dimethylamino-1-buten (93 %ig ; 0,04 Mol) werden unter Rühren im Verlauf von 20 Minuten 34,5 g einer 23,0 %igen Rohlösung des 2-Chlor-5-nitrophenylisocyanates in Chlorbenzol (ca. 25 ml ; 0,04 Mol) bei Raumtemperatur zugetropft. Es wird 5 Minuten nachgerührt. Dann werden 10 ml Wasser hinzugefügt und unter starkem Rühren ca. 3,9 ml conc. HCl so zugetropft, daß ein pH-Wert von 5 nicht unterschritten und zum Schluß gehalten wird. Dies nimmt ca. 1,5 bis 2 Stunden in Anspruch. Es wird noch 1/2 Stunde nachgerührt. Das ausgefallene Produkt wird abgesaugt, die organische Phase auf ca. 10 ml eingeengt und auf − 15 °C abgekühlt, wobei weiteres Produkt ausfällt. Nach dem Trocknen im Vakuum bei 50 °C bleiben 10,6 g (91 % der theoretischen Ausbeute) Pivaloyl-2-chlor-5-nitroacetanilid mit einem Schmelzpunkt von 116 bis 119 °C.

## Beispiel 3

Zu einer Lösung von 145 ml (207 g, 2,1 Mol) Phosgen in 800 ml Chlorbenzol werden bei 0 °C unter gutem Rühren im Laufe von ca. 30 Minuten 180 g (1,0 Mol) 2-Chlor-5-nitroanilin (96 %ig) als Feststoff eingetragen. Die gelb-braune Suspension wird auf 55 °C aufgeheizt. Dabei wird ein schwacher Strom Phosgen eingeleitet, so daß überschüssiges Phosgen am Rückfluß siedet. Unter HCl-Entwicklung klärt sich die Suspension im Laufe von 4 Stunden zur Lösung. Es wird noch eine Stunde nachphosgeniert.

Überschüssiges Phosgen und Chlorbenzol werden bis auf ein Restvolumen von 600 ml abdestilliert. Es entsteht eine etwa 23 gew.-%ige Lösung des 2-Chlor-5-nitro-phenyl-isocyanats in Chlorbenzol, die einen Phosgengehalt von weniger als 0,05 % besitzt.

Gehalt nach Titration : 188,3 g Isocyanat = 95 % der Theorie.

Bei der Destillation über eine beheizbare Kolonne werden bei einer Kopftemperatur von etwa 130 °C und einem Druck von 1 mbar 183 g 2-Chlor-5-nitro-phenylisocyanat (99 %ig) erhalten. Dies entspricht 93 % der theoretischen Ausbeute. Der Schmelzpunkt des Produktes beträgt 93-95 °C.

## Beispiel 4

Zu einer gerührten Lösung von 75,8 g 3,3-Dimethyl-2-dimethylamino-1-buten (94 %ig, 0,56 mol) in 300 g (270 ml) trockenem Chlorbenzol werden im Verlauf von ca. 30 Min. bei Raumtemperatur 480 g der Rohlösung des 2-Chlor-5-nitro-phenylisocyanats nach Beispiel 3 (21,1 %ig, 0,56 mol, 350 ml) zugetropft. Es wird 10 Min. nachgerührt, der Tropftrichter durch ein Gaseinleitungsrohr ersetzt und bei Raumtemperatur trockener Chlorwasserstoff eingeleitet. Durch Kühlen wird eine Temperatur von ca. 20 °C gehalten. Nach 2-3 Min. beginnt das Immoniumchlorid des β-Dimethylamino-γ,γ-dimethylpenten(2)-carbonsäure-(2-chlor-5-nitro)-anilids auszufallen. Der entstehende Brei wird zunehmend dickflüssiger, kann aber gut gerührt werden. Wenn kein HCl mehr aufgenommen wird, wird über eine Glasfritte abgesaugt und zweimal mit jeweils 100 ml trockenem Chlorbenzol gewaschen. Der Filterkuchen auf der Fritte wird nun mit 150 ml Wasser versetzt und gerührt. Nach 3-5 Min. gent das Immoniumsalz in Lösung.

Die Temperatur steigt dabei auf 35 °C an. Es bilden sich zwei klare Phasen, eine wässrige, Immoniumsalzhaltige untere Phase und eine obere Phase aus festgehaltenem Chlorbenzol. Nun wird unter kräftigem Rühren tropfenweise mit konzentrierter Natronlauge versetzt, bis ein pH-Wert von etwa 5 erreicht ist (ca. 40 ml). Dabei steigt die Temperatur auf etwa 65 °C an und aus der Chlorbenzolphase beginnt das Pivaloyl-2-chlor-5-nitro-acetanilid zu kristallisieren. Während der Neutralisation findet ein Phasenwechsel statt. Die jetzt das Produkt enthaltende organische Phase sinkt nach unten, die wässrige Dimethylammoniumchlorid-haltige Phase setzt sich oben ab. Es wird noch 3 Stunden nachgerührt und schließlich auf 0 °C abgekühlt. Jetzt wird abgesaugt und zweimal mit jeweils 100 ml Wasser gewaschen. Die Chlorbenzolphase kann direkt oder nach Ausfällung von restlichem Pivaloyl-2-chlor-5-nitro-acetanilid durch Abkühlen auf – 15 °C in die Hydrolyse des nächsten Ansatzes zurückgeführt werden, da sie außer dem Pivaloyl-2-chlor-5-nitro-acetanilid keine weitere Verunreinigungen enthält. Auf diese Weise wurde nach Trocknung 150-160 g Pivaloyl-2-chlor-5-nitro-acetanilid mit einem Schmelzpunkt von 118-120 °C erhalten. Dies entspricht 90-95 % der theoretischen Ausbeute.

**Ansprüche**

1. 3-Nitro-pivaloyl-acetanilide der Formel

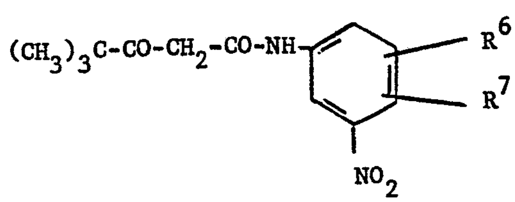

in der

$R^6$ und $R^7$ unabhängig voneinander für Wasserstoff, Methyl, Ethyl, Methoxy, Methylthio, Chlor, Brom, Nitro, Dimethylamino, Diethylamino oder Benzyl-methyl-amino stehen.

2. 2-Chlor-5-nitro-pivaloyl-acetanilid.

3. Verfahren zur Herstellung von 3-Nitro-pivaloyl-acetaniliden der Formel

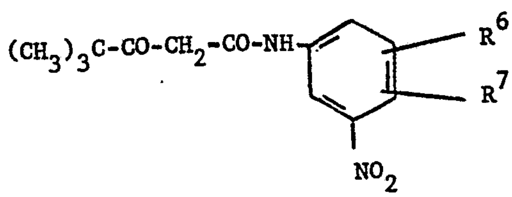

in der

$R^6$ und $R^7$ unabhängig voneinander für Wasserstoff, Methyl, Ethyl, Methoxy, Methylthio, Chlor, Brom, Nitro, Dimethylamino, Diethylamino oder Benzyl-methyl-amino stehen,

dadurch gekennzeichnet, daß man einen Pivaloylessigsäureester der Formel

$$(CH_3)_3C{-}CO{-}CH_2{-}COR^9$$

in der

$R^9$ Methoxy oder Ethoxy bedeutet,

mit 3-Nitroanilinen der Formel

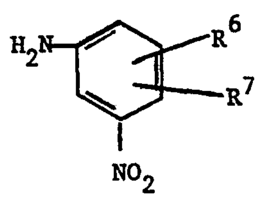

in der

$R^6$ und $R^7$ die oben angegebene Bedeutung haben,

bei einer Temperatur von 50 bis 200 °C, gegebenenfalls in Gegenwart eines inerten Verdünnungsmittels, kondensiert.

4. Verfahren nach Anspruch 3, dadurch gekennzeichnet, daß man ein Verdünnungsmittel einsetzt, das mit dem bei der Kondensation abgespaltenen Alkohol ein Azeotrop bildet.

5. Verfahren zur Herstellung von 3-Nitro-pivaloyl-acetaniliden der Formel

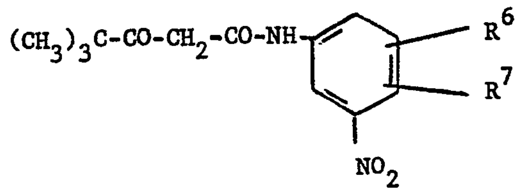

in der

$R^6$ und $R^7$ unabhängig voneinander für Wasserstoff, Methyl, Ethyl, Methoxy, Methylthio, Chlor, Brom, Nitro, Dimethylamino, Diethylamino oder Benzyl-methyl stehen,

dadurch gekennzeichnet, daß man ein Enamin der Formel

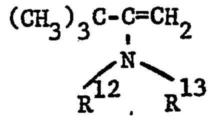

in der

$R^{12}$ und $R^{13}$ unabhängig voneinander $C_1$-$C_4$-Alkyl, Benzyl oder Phenyl bedeuten oder $R^{12}$ und $R^{13}$ gemeinsam mit dem Stickstoffatom, das sie substituieren, einen 5- bis 7-gliedrigen stickstoffhaltigen Heterocyclus bedeuten, der gegebenenfalls weitere Heteroatome enthalten kann,

mit einem 3-Nitro-phenylisocyanat der Formel

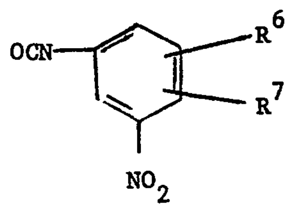

in der

$R^6$ und $R^7$ die angegebene Bedeutung haben,

bei einer Temperatur von − 5 °C bis + 100 °C, gegebenenfalls in Gegenwart eines inerten Verdünnungsmittels, so umsetzt, daß das Enamin stets im Überschuß vorliegt, und das als Zwischenprodukt entstehende β-Amino-γ,γ-dimethyl-penten-(2)-carbonsäure-3-nitro-anilid der Formel

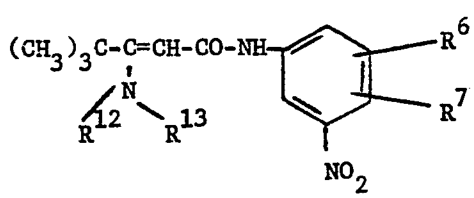

in der

$R^6$, $R^7$, $R^{12}$ und $R^{13}$ die angegebene Bedeutung haben,

gegebenenfalls nach vorheriger Isolierung und Reinigung, hydrolisiert.

6. Verfahren nach Anspruch 5, dadurch gekennzeichnet, daß man die Hydrolyse durch Zugabe von mindestens der stöchiometrisch erforderlichen Menge Wasser und Einhalten eines pH-Bereichs von 4 bis 6 durch dosierte Zugabe von wäßriger Salzsäure durchführt.

7. Verfahren nach Anspruch 5, dadurch gekennzeichnet, daß man das Zwischenprodukt durch Einleiten von gasförmigem Chlorwasserstoff in das Reaktionsgemisch als Immoniumsalz ausfällt und nach teilweiser oder vollständiger Entfernung des Lösungsmittels durch Verrühren mit Wasser zum Endprodukt hydrolisiert.

8. β-amino-γ,γ-dimethyl-penten-(2)-carbonsäure-3-nitroanilide der Formel

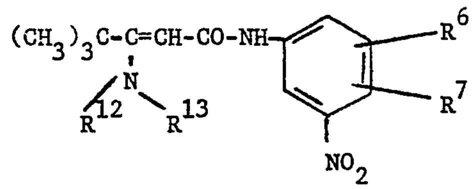

in der
$R^6$, $R^7$, $R^{12}$ und $R^{13}$ die in Anspruch 5 gegebene Bedeutung haben, in freier Form oder in Form ihrer Immoniumsalze der Salzsäure der Formel

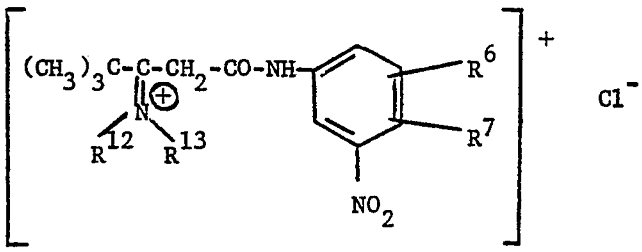

in der
$R^6$, $R^7$, $R^{12}$ und $R^{13}$ die angegebene Bedeutung haben.

9. Verwendung der 3-Nitro-pivaloyl-acetanilide nach Anspruch 1 zur Synthese von Kupplungskomponenten für die Farbfotografie der Formel

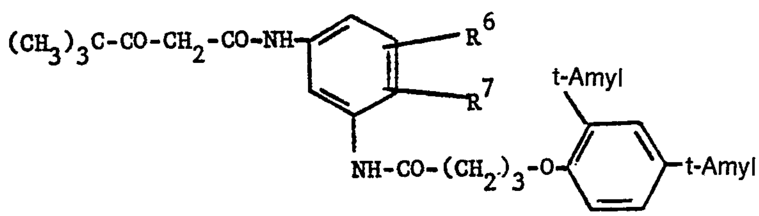

in der
$R^6$ und $R^7$ unabhängig voneinander für Wasserstoff, Methyl, Ethyl, Methoxy, Methylthio, Chlor, Brom, Nitro, Dimethylamino, Diethylamino oder Benzyl-methyl-amino stehen,
durch Reduktion der Nitrogruppe des 3-Nitro-pivaloylacetanilids zur Aminogruppe und anschließende Umsetzung dieser Aminogruppe mit 2,4-Di-tert.-amyl-phenoxybuttersäurehalogenid unter Abspaltung von Halogenwasserstoff.

**Claims**

1. 3-Nitro-pivaloyl-acetanilides of the formula

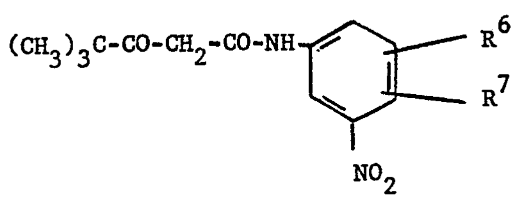

in which
$R^6$ and $R^7$ independently of one another represent hydrogen, methyl, ethyl, methoxy, methylthio, chlorine, bromine, nitro, dimethylamino, diethylamino or benzyl-methyl-amino.

2. 2-Chloro-5-nitro-pivaloyl-acetanilide.

3. Process for the preparation of 3-nitro-pivaloyl-acetanilides of the formula

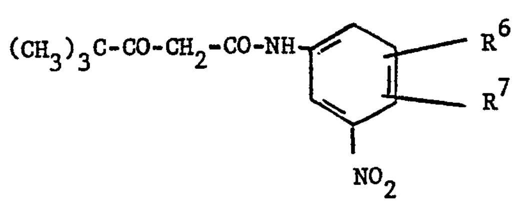

in which

$R^6$ and $R^7$ independently of one another represent hydrogen, methyl, ethyl, methoxy, methylthio, chlorine, bromine, nitro, dimethylamino, diethylamino or benzyl-methyl-amino,

characterised in that a pivaloylacetic acid ester of the formula

$$(CH_3)_3C—CO—CH_2—COR^9$$

in which

$R^9$ denotes methoxy or ethoxy,

is condensed with 3-nitroanilines of the formula

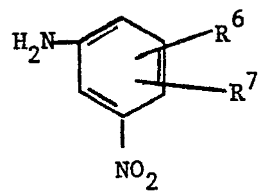

in which

$R^6$ and $R^7$ have the abovementioned meaning, at a temperature of 50 to 200 °C, if appropriate in the presence of an inert diluent.

4. Process according to Claim 3, characterised in that a diluent is used which forms an azeotrope with the alcohol which is split off during the condensation.

5. Process for the preparation of 3-nitro-pivaloyl-acetanilides of the formula

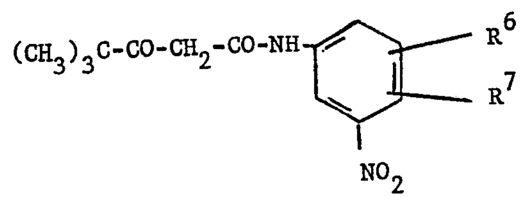

in which

$R^6$ and $R^7$ independently of one another represent hydrogen, methyl, ethyl, methoxy, methylthio, chlorine, bromine, nitro, dimethylamino, diethylamino of benzyl-methyl,

characterised in that an enamine of the formula

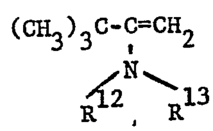

in which

$R^{12}$ and $R^{13}$ independently of one another denote $C_1$-$C_4$-alkyl, benzyl or phenyl, or $R^{12}$ and $R^{13}$, together with the nitrogen atom on which they are substituents, denote a 5-membered to 7-membered nitrogen-containing heterocyclic ring which can optionally contain further hetero-atoms,

is reacted with a 3-nitro-phenyl isocyanate of the formula

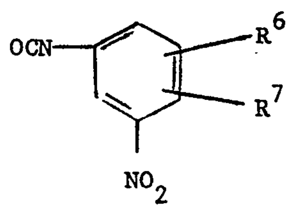

in which

$R^6$ and $R^7$ have the meaning indicated,

at a temperature of $- 5$ °C to $+ 100$ °C, if appropriate in the presence of an inert diluent, in such a way that the enamine is always present in excess, and the β-amino-γ,γ-dimethylpent-2-ene-carboxylic acid 3-nitroanilide formed as an intermediate, of the formula

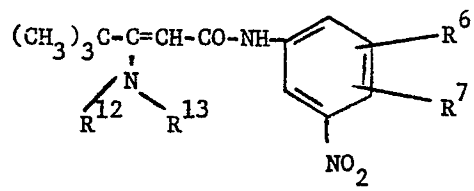

in which

$R^6$, $R^7$, $R^{12}$ and $R^{13}$ have the meaning indicated, is hydrolysed, if appropriate after prior isolation and purification.

6. Process according to Claim 5, characterised in that the hydrolysis is carried out by adding at least the stoichiometrically required amount of water and maintaining a pH range of 4 to 6 by metering in aqueous hydrochloric acid.

7. Process according to Claim 5, characterised in that the intermediate is precipitated as the immonium salt by passing gaseous hydrogen chloride into the reaction mixture and, after partial or total removal of the solvent, is hydrolysed to the end product by stirring with water.

8. β-Amino-γ,γ-dimethyl-pent-2-ene-carboxylic acid 3-nitro-anilides of the formula

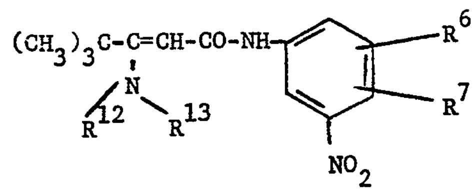

in which

$R^6$, $R^7$, $R^{12}$ and $R^{13}$ have the meaning given in Claim 5, in the free form or in the form of their immonium salts with hydrochloric acid, of the formula

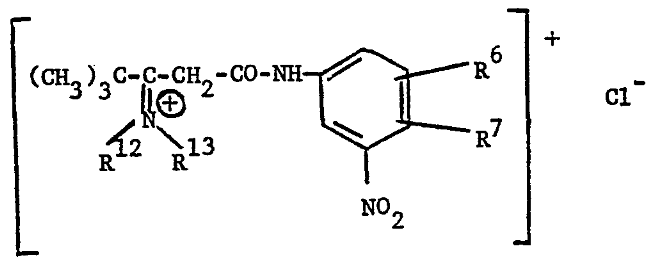

in which

$R^6$, $R^7$, $R^{12}$ and $R^{13}$ have the meaning indicated.

9. Use of the 3-nitro-pivaloyl-acetanilides according to Claim 1 for the synthesis of coupling components for colour photography, of the formula

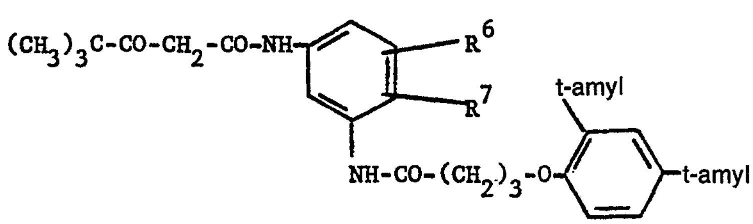

in which

$R^6$ and $R^7$ independently of one another represent hydrogen, methyl, ethyl, methoxy, methylthio, chlorine, bromine, nitro, dimethylamino, diethylamino or benzyl-methyl-amino,

by reduction of the nitro group of 3-nitro-pivaloyl-acetanilide to give the amino group and subsequent reaction of this amino group with 2,4-di-tert.-amyl-phenoxybutyryl halide, hydrogen halide being split off.

**Revendications**

1. 3-nitro-pivaloyl-acétanilides de formule

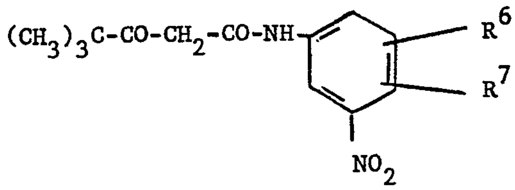

dans laquelle

$R^6$ et $R^7$ représentent, indépendamment l'un de l'autre, un atome d'hydrogène, un groupe méthyle, éthyle, méthoxy, méthylthio, un atome de chlore ou de brome, un groupe nitro, diméthylamino, diéthylamino ou benzyl-méthyl-amino.

2. Le 2-chloro-5-nitro-pivaloyl-acétanilide.

3. Procédé de préparation de 3-nitro-pivaloyl-acétanilides de formule

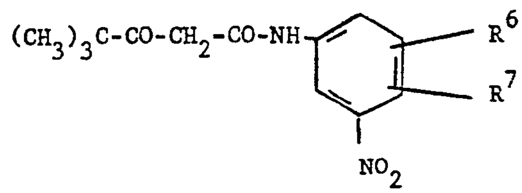

dans laquelle

$R^6$ et $R^7$ représentent, indépendamment l'un de l'autre, un atome d'hydrogène, un groupe méthyle, éthyle, méthoxy, méthylthio, un atome de chlore ou de brome, un groupe nitro, diméthylamino, diéthylamino ou benzyl-méthyl-amino,

caractérisé en ce qu'on condense à une température de 50 à 200 °C, éventuellement en présence d'un diluant inerte, un ester d'acide pivaloylacétique de formule

$$(CH_3)_3C—CO—CH_2—COR^9$$

dans laquelle

$R^9$ représente un groupe méthoxy ou éthoxy

avec des 3-nitroanilines de formule

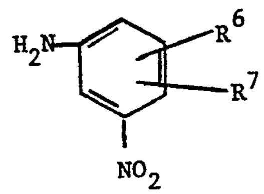

dans laquelle

$R^6$ et $R^7$ ont le sens indiqué ci-dessus.

4. Procédé selon la revendication 3, caractérisé en ce qu'on utilise un diluant qui forme un azéotrope avec l'alcool scindé lors de la condensation.

5. Procédé de préparation de 3-nitro-pivaloyl-acétanilides de formule

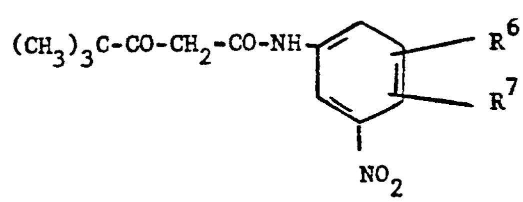

dans laquelle

$R^6$ et $R^7$ représentent, indépendamment l'un de l'autre, un atome d'hydrogène, un groupe méthyle, éthyle, méthoxy, méthylthio, un atome de chlore ou de brome, un groupe nitro, diméthylamino, diéthylamino ou benzyl-méthyle,

caractérisé en ce qu'on fait réagir une énéamine de formule

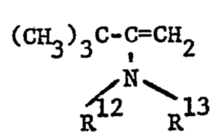

dans laquelle

$R^{12}$ et $R^{13}$ représentent, indépendamment l'un de l'autre, un groupe alkyle en $C_1$-$C_4$, benzyle ou phényle, ou bien $R^{12}$ et $R^{13}$, pris avec l'atome d'azote sur lequel ils sont fixés comme substituants, forment un hétérocycle azote pentagonal à heptagonal, qui peut éventuellement comporter d'autres hétéroatomes,

avec un isocyanate de 3-nitro-phényle de formule

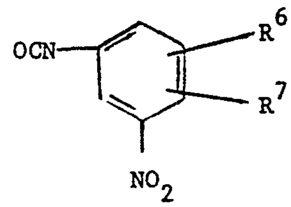

dans laquelle

$R^6$ et $R^7$ ont le sens indiqué,

à une température de – 5 °C à 100 °C, éventuellement en présence d'un diluant inerte, de manière que l'énéamine soit toujours présente en excès, et, éventuellement après un isolement et une purification préalables, on hydrolyse le 3-nitro-anilide de l'acide β-amino-γ,γ-diméthyl-pentène-(2)-carboxylique de formule

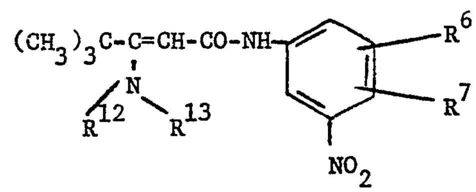

dans laquelle

$R^6$, $R^7$, $R^{12}$ et $R^{13}$ ont le sens indiqué,

obtenu comme produit intermédiaire.

6. Procédé selon la revendication 5, caractérisé en ce qu'on effectue l'hydrolyse par addition d'au moins la quantité stœchiométriquement nécessaire d'eau et maintien d'un pH de 4 à 6 par addition dosée d'acide chlorhydrique aqueux.

7. Procédé selon la revendication 5, caractérisé en ce qu'on précipite le produit intermédiaire sous forme de sel d'immonium, par introduction de gaz chlorhydrique dans le mélange réactionnel, et en ce que, après élimination partielle ou totale du solvant, on hydrolyse par délayage à l'eau pour obtenir le produit final.

8. 3-nitro-anilides d'acides β-amino-γ,γ-diméthyl-pentène-(2)-carboxyliques de formule

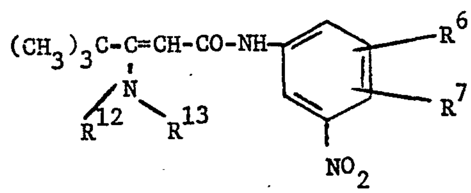

dans laquelle

$R^6$, $R^7$, $R^{12}$ et $R^{13}$ ont le sens indiqué à la revendication 5, sous forme libre ou sous forme de leurs sels d'immonium de l'acide chlorhydrique, répondant à la formule

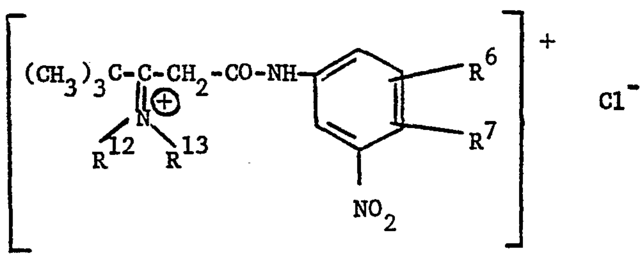

dans laquelle

$R^6$, $R^7$, $R^{12}$ et $R^{13}$ ont le sens indiqué.

9. Utilisation des 3-nitro-pivaloyl-acétanilides selon la revendication 1 pour la synthèse de copulants pour la photographie en couleurs, qui répondent à la formule

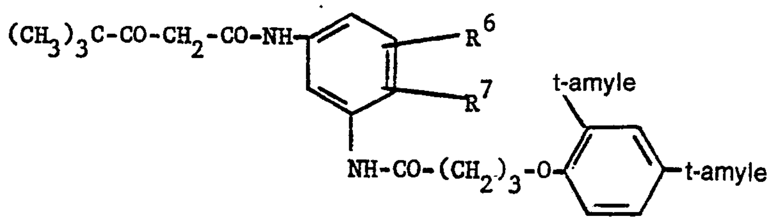

dans laquelle

$R^6$ et $R^7$ représentent, indépendamment l'un de l'autre, un atome d'hydrogène, un groupe méthyle, éthyle, méthoxy, méthylthio, un atome de chlore ou de brome, un groupe nitro, diméthylamino, diéthylamino ou benzyl-méthyl-amino,
par réduction du groupe nitro du 3-nitro-pivaloyl-acétanilide en groupe amino puis réaction de ce groupe amino avec un halogénure de l'acide 2,4-di-tertio-amyl-phénoxy-butyrique avec scission de l'hydracide halogéné (halogénure d'hydrogène).